## Europäisches Patentamt
## European Patent Office
## Office européen des brevets

(11) Veröffentlichungsnummer: **0 085 372**
**A1**

(12) **EUROPÄISCHE PATENTANMELDUNG**

(21) Anmeldenummer: 83100570.7

(51) Int. Cl.³: **C 07 C 29/82**
**C 07 C 31/10**

(22) Anmeldetag: 22.01.83

(30) Priorität: 01.02.82 DE 3203262

(43) Veröffentlichungstag der Anmeldung:
10.08.83 Patentblatt 83/32

(84) Benannte Vertragsstaaten:
BE DE FR GB IT NL

(71) Anmelder: BASF Aktiengesellschaft
Carl-Bosch-Strasse 38
D-6700 Ludwigshafen(DE)

(72) Erfinder: Fischer, Karl, Dr.
Luginsland 22
D-6520 Worms(DE)

(72) Erfinder: Strohmeyer, Max, Dr.
Woogstrasse 41
D-6703 Limburgerhof(DE)

(72) Erfinder: Reutemann, Werner
Vom-Stein-Strasse 2 C
D-6712 Bobenheim-Roxheim(DE)

(72) Erfinder: Weber, Willi Albert
Bauhausstrasse 53
D-6700 Ludwigshafen(DE)

(54) Verfahren zur kontinuierlichen destillativen Gewinnung von Propanol.

(57) Kontinuierliche destillative Gewinnung von Propanol aus wasserhaltigem Rohpropanol indem man das Wasser bei der Destillation mittels Dipropylether in Form eines propanolhaltigen Wasser/Dipropylether-Azeotrops über Kopf entfernt, einen Teil des Dipropylethers nach Kondensation und Phasentrennung des azeotropen Gemisches in die Destillationskolonne zurückführt, die hochsiedenden Bestandteile als Sumpfprodukt entfernt und das Propanol der Kolonne als dampfförmigen Seitenabzug entnimmt.

P = Propanol
W = Wasser
E = Dipropylether
HS = Hochsieder

EP 0 085 372 A1

Verfahren zur kontinuierlichen destillativen Gewinnung von Propanol

Die vorliegende Erfindung betrifft ein neues Verfahren zur kontinuierlichen destillativen Gewinnung von Propanol aus wasserhaltigem Rohpropanol.

Propanol wird großtechnisch hauptsächlich durch Hydroformylierung von Ethylen und anschließende Hydrierung des zunächst entstehenden Propionaldehydes hergestellt (vgl. Ullmanns Enzyklopädie der Technischen Chemie, 4. Auflage, 1980, Band 1, S. 445-446).

Da die Hydrierung in Gegenwart von Wasser vorgenommen wird, um der Bildung von Dipropylether entgegenzuwirken, enthält das Rohpropanol neben etwa 0,2 bis 5 Gew.% hochsiedenden Bestandteilen und 0,2 bis 5 Gew.% Dipropylether 2 bis 20 Gew.% Wasser.

Die technisch wesentliche Aufgabe bei der Aufarbeitung des Rohpropanols, die in der Abtrennung des Wassers besteht, wurde bisher auf die Weise gelöst (siehe Ullmann cit.), daß man ein Schleppmittel wie Cyclohexan mitverwendet, mit dessen Hilfe zunächst das Wasser in Form eines Wasser/Schleppmittel-Azeotrops abgetrennt wird, welches außerdem noch etwas (etwa 5 bis 15 Gew.% der Wasserphase) Propanol enthält. In weiteren Destillationsschritten werden sodann der Dipropylether und die hochsiedenden Bestandteile zur Gewinnung von Reinpropanol abgetrennt.

Diese Arbeitsweise ist wegen der Mitverwendung des systemfremden Schleppmittels sowie vor allem wegen des erforderlichen apparativen Aufwandes von mehreren hintereinandergeschalteten Kolonnen wirtschaftlich nachteilig.

Mi/P

0085372

Der Erfindung lag daher die Aufgabe zugrunde, diese Nachteile zu beheben.

Demgemäß wurde ein Verfahren zur kontinuierlichen destillativen Gewinnung von Propanol aus wasserhaltigem Rohpropanol gefunden, welches dadurch gekennzeichnet ist, daß man das Wasser bei der Destillation mittels Dipropylether in Form eines propanolhaltigen Wasser/Dipropylether-Azeotropes über Kopf entfernt, einen Teil des Dipropylethers nach Kondensation und Phasentrennung des azeotropen Gemisches in die Destillationskolonne zurückführt, die hochsiedenden Bestandteile als Sumpfprodukt entfernt und das Propanol der Kolonne als dampfförmigen Seitenabzug entnimmt.

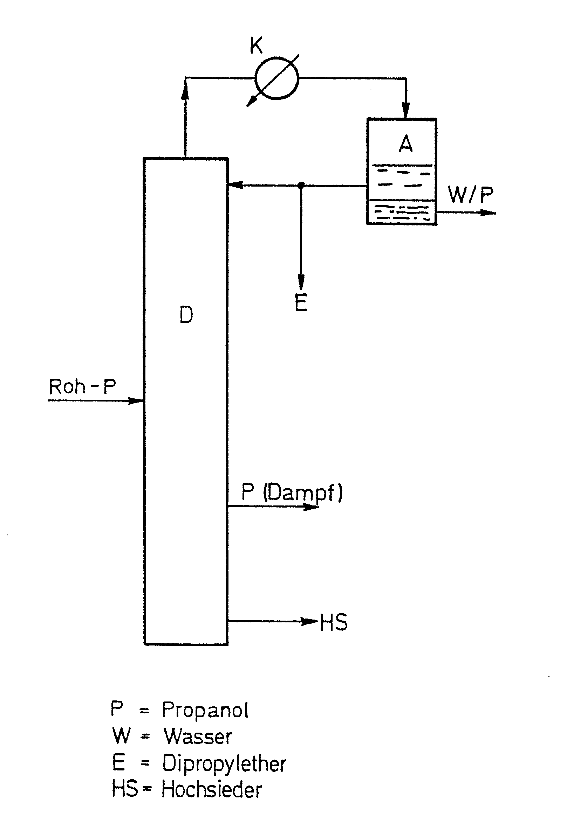

Eine bewährte Ausführungsform des Verfahrens sei anhand der Zeichnung näher erläutert.

Läßt man eine eventuelle Feinreinigung des gewonnenen Propanols außer Betracht, so findet das gesamte Trennverfahren in einer einzigen Kolonne, der Kolonne D, statt. Auf die Bauart der Kolonne kommt es nicht an, d.h. man kann Glockenböden-, Siebböden- oder Ventilbödenkolonnen sowie auch Füllkörperkolonnen verwenden, jedoch empfiehlt sich zumindest für den unteren Bereich die Ausgestaltung als Bodenkolonne, da in diesem Falle die Abtrennung der dampfförmigen Propanolphase von der Flüssigphase zuverlässiger gelingt. Vorzugsweise enthält die Kolonne D 10 bis 50 theoretische Böden.

Man führt das Rohpropanol dampfförmig oder flüssig in den mittleren Bereich der Kolonne, also etwa auf Höhe des 6. bis 30. Bodens, von unten gezählt.

Zur vollständigen azeotropen Entfernung des Wassers sind bei Normaldruck pro Gew.% etwa 6 bis 8 Gew.% Dipropylether erforderlich. Ist zu Beginn der Destillation weniger Dipropylether zugegen, ist es zweckmäßig, die fehlende Menge gesondert zuzufügen. Das Rohpropanol enthält in aller Regel, besonders wenn es der Hydroformylierung von Ethylen und der anschließenden Hydrierung des Propionaldehyds entstammt, etwa 0,2 bis 5 Gew.% Dipropylether. Es muß daher soviel des Ethers fortlaufend aus dem System entfernt werden, wie mit dem Rohpropanol neu eingeführt wird.

Das am Kopf der Kolonne entweichende Azeotrop wird im Kühler K kondensiert und im Abscheider A in eine Phase, die überwiegend aus dem Dipropylether besteht, und eine wäßrige Phase zerlegt, die etwa 10 Gew.% Propanol enthält.

Da für die Hydrierung des Propionaldehyds Wasser benötigt wird, führt man die wäßrige Phase zweckmäßigerweise in die Hydrierstufe zurück. Das hierin enthaltene Propanol stört die Hydrierung naturgemäß nicht.

Die Etherphase wird nach Maßgabe der für die Azeotrop- -Destillation erforderlichen Menge zum Teil nach D zurückgeführt und dem System zum restlichen Teil entnommen. Der Dipropylether eignet sich gut als Komponente für Lösungsmittelgemische und kann in der anfallenden Form ohne weitere Reinigung für diesen Zweck verwendet werden.

Die hochsiedenden Substanzen, die hauptsächlich aus Kondensationsprodukten des Propionaldehyds und deren Ethern bestehen, werden der Kolonne D als Sumpfprodukt entnommen. Da sie sich chemisch weitgehend inert verhalten, kann man sie als Reaktionsmedium für chemische Reaktionen, z.B. für die Hydroformylierung verwenden.

Auf Höhe etwa des 4. bis 15. Bodens wird das Propanol als dampfförmiger Seitenabzug aus der Kolonne abgeführt. Dieses Produkt enthält nur noch wenig hochsiedende Substanzen als Begleitstoffe (etwa 0,005 bis 0,5 Gew.%), so daß es für die meisten Verwendungszwecke bereits rein genug ist. Gewünschtenfalls kann man dieses Propanol noch einer üblichen destillativen Feinreinigung unterziehen, wofür man eine Kolonne mit 10 bis 50 theoretischen Böden verwendet.

Man kann das erfindungsgemäße Destillationsverfahren bei 0,2 bis 5 bar vornehmen, arbeitet in der Regel jedoch bei Normaldruck. Aus dem angewandten Druck ergeben sich die entsprechenden Betriebstemperaturen der Kolonne (bei Normaldruck ca. $115^{\circ}C$ im Sumpf und ca. $85^{\circ}C$ am Kopf). Für den Kolonnenrücklauf empfiehlt sich ein Verhältnis von 5:1 bis 10:1.

Das Verfahren, dessen wesentliches Merkmal die azeotrope Abtrennung des Wassers vom Rohpropanol mittels Dipropylether ist, eignet sich selbstverständlich nicht nur für die Aufarbeitung von Rohpropanol, welches der Hydroformylierung von Ethylen und der anschließenden Hydrierung des Propionaldehyds entstammt, sondern auch für die Gewinnung von Propanol aus dessen wasserhaltigen Gemischen beliebiger Herkunft.

Beispiel

In die Mitte einer Kolonne von 11 m Höhe, 0,3 m Innendurchmesser und 34 theoretischen Böden wurden stündlich 200 kg Rohpropanol gegeben, welches der Hydroformylierung von Ethylen und der anschließenden Hydrierung des Propionaldehyds an einem Ni-Kontakt in Gegenwart von 14 kg/h Wasser entstammte.

Dieses Rohpropanol setzte sich aus folgenden wesentlichen Bestandteilen zusammen:

183,0 kg (91,5 Gew.%) Propanol
14,0 kg ( 7,0 Gew.%) Wasser
1,3 kg ( 0,7 Gew.%) Dipropylether
<u>1,7 kg ( 0,8 Gew.%) hochsiedende Substanzen</u>
200   kg (100  Gew.%)

In der Destillationsanlage, welche der Zeichnung entsprach, befanden sich etwa 100 kg Dipropylether. Die Kolonne wurde mit einem Rücklaufverhältnis von etwa 8:1 betrieben. Nach der Phasentrennung im Abscheider A fielen pro Stunde 15,3 kg einer wäßrigen Phase an, die 1,3 kg Propanol enthielt. Diese Phase wurde in die Hydrierstufe zurückgeführt. Weiterhin wurde durch Leitung E pro Stunde eine organische Phase aus 1,3 kg Dipropylether, 0,4 kg Propanol und geringen Mengen Wasser abgezogen.

Aus dem Sumpf der Kolonne wurden stündlich 1,7 kg hochsiedende Substanzen zusammen mit 0,9 kg Propanol entfernt, und auf der Höhe des 8. Bodens fielen stündlich 180,4 kg praktisch reines Propanol als Verfahrensprodukt an. Dies entspricht einer Destillationsausbeute von 98,6 % des eingesetzten Propanols.

## Patentanspruch

Verfahren zur kontinuierlichen destillativen Gewinnung von Propanol aus wasserhaltigem Rohpropanol, <u>dadurch gekenn-zeichnet</u>, daß man das Wasser bei der Destillation mittels Dipropylether in Form eines propanolhaltigen Wasser/Dipropylether-Azeotrops über Kopf entfernt, einen Teil des Dipropylethers nach Kondensation und Phasentrennung des azeotropen Gemisches in die Destillationskolonne zurückführt, die hochsiedenden Bestandteile als Sumpfprodukt entfernt und das Propanol der Kolonne als dampfförmigen Seitenabzug entnimmt.

Zeichn.

P  =  Propanol
W  =  Wasser
E  =  Dipropylether
HS =  Hochsieder

# EUROPÄISCHER RECHERCHENBERICHT

**0085372**
Nummer der Anmeldung

Europäisches
Patentamt

## EINSCHLÄGIGE DOKUMENTE

| Kategorie | Kennzeichnung des Dokuments mit Angabe. soweit erforderlich. der maßgeblichen Teile | Betrifft Anspruch | KLASSIFIKATION DER ANMELDUNG (Int. Cl ³) |
|---|---|---|---|
| X | US-A-2 489 619 (C.S. CARLSON et al.) <br> * Spalte 3, Zeile 70 - Spalte 4, Zeile 21; Ansprüche 1,5,7 * | 1 | C 07 C  29/82 <br> C 07 C  31/10 |
| X | L.H. HORSLEY et al.: "Azeotropic data III", 1973, Seiten 464-466, American Chemical Society, Washington D.C., USA <br> * Seite 466, Zeile 14 - Seite 467, Zeile 14, Nr. 16096; Seite 464, Zeile 20 - Seite 465, Zeile 20, Nr. 16077 * | 1 | |
| Y | US-A-2 640 017 (A.R. GRAFF) <br> * Spalte 1, Zeilen 30-53; Spalte 2, Zeilen 10-13; Spalte 2, Zeilen 14-32; Abbildung; Ansprüche * | 1 | RECHERCHIERTE SACHGEBIETE (Int. Cl. ³) |
| Y | US-A-3 528 891 (K. RAUCH et al.) <br> * Spalte 2, Zeilen 52-64; Ansprüche; Spalte 4, Zeilen 4-15; Abbildung * | 1 | C 07 C  29/00 |
| Y | CHEMICAL ABSTRACTS, Band 67, Nr. 10, 4. September 1967, Seite 4260, Nr. 45131t, Columbus, Ohio, USA <br> IKUHO YAMADA et al.: "Azeotropic distillation design" & KAGAKU KOGAKU 31(4), 395-398, 1967 * Zusammenfassung * | 1 | |

--- -/-

Der vorliegende Recherchenbericht wurde für alle Patentansprüche erstellt

| Recherchenort DEN HAAG | Abschlußdatum der Recherche 21-04-1983 | Prüfer DELHOMME H.A. |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | FR-A- 577 169 (SOCIETE RICARD ALLENET) <br> * Abbildung * <br><br> ----- | 1 | |

**RECHERCHIERTE SACHGEBIETE (Int Cl ³)**

Der vorliegende Recherchenbericht wurde für alle Patentansprüche erstellt.

| Recherchenort <br> DEN HAAG | Abschlußdatum der Recherche <br> 21-04-1983 | Prüfer <br> DELHOMME H.A. |
|---|---|---|